# EUROPEAN PATENT SPECIFICATION

(11) **EP 2 233 085 B1**
(45) Date of publication and mention of the grant of the patent: **16.05.2012**
(21) Application number: 09004115.3
(22) Date of filing: 23.03.2009
(51) Int. Cl.: A61B 17/14, B27B 33/02, B23D 61/12

(54) **Surgical instrument for cutting bone**
Chirurgisches Instrument zum Schneiden von Knochen
Instrument chirurgical pour couper les os

(43) Date of publication of application: 29.09.2010
(73) Proprietor: Mectron S.p.A., 16042 Carasco GE (IT)
(72) Inventor: Bianchetti, Fernando, 16043 Chiavarie(GE) (IT); Vercellotti, Domenico, 16039 Sestri Levante (GE) (IT)
(74) Representative: Hauck Patent- und Rechtsanwälte

(56) References cited:
- WO-A-93/01751
- WO-A-2007/020831
- FR-A- 2 656 788
- US-A1- 2005 113 840
- US-A1- 2006 272 468

## Description

The present invention is directed to a piezoelectric surgical instrument for cutting bone, which is adapted to be fixed at a handpiece, said instrument comprising an instrument shaft adapted to be fixed at the handpiece and a joining instrument tip which has at its free end a plurality of adjacent cutting teeth with two tooth flanks merging into a tooth tip and emanating from a tooth root.

Such a surgical instrument is known from WO 93/01751. In order to cut bone the instrument tip is oscillated so that it vibrates in the ultrasonic frequency range. With such an instrument precise cuts in bone tissue can be carried out. The piezoelectric surgery is based on the principle of the ultrasonic technique. Herewith, a micrometric cutting action in the non-visible range of 60-200 µm as well as a selective cutting action can be obtained, i.e. mineralized hard tissue is cut while soft tissue is spared. Accordingly, an especially precise cutting action, the careful treatment of important anatomic structures and a bloodless surgical field can be obtained. The piezoelectric surgery is especially used in the oral field, for instance for cutting jawbone.

Accordingly, the present invention is directed to a piezoelectric bone scalpel which can be used in the field of implantology. The tip of the instrument oscillates with a modulatable ultrasonic frequency. The instrument has the advantage that it cuts only hard material so that anatomic soft tissue structures are spared.

For instance, the above-described surgical instrument for cutting bone is used for osteotomy techniques in the upper and lower jawbone, as crest expansion, removing of bone blocks.

The known instrument has an instrument shaft adapted to be mounted at a handpiece and merging into an instrument tip flattened with respect to the shaft and enlarged at its free end and having five adjacent cutting teeth. The tooth flanks of all the cutting teeth have the same slope.

When handling the instrument the same is normally put with the tip, i.e. the cutting teeth, downwardly onto the bone which is to be cut, for instance onto the crest of the jawbone. For carrying out the desired cut the instrument tip is oscillated wherein the same oscillates especially forwardly and backwardly with a frequency lying in the ultrasonic frequency range.

Since the bones, especially jawbones, which are to be cut normally have no homogenous bone structure (hard and soft areas alternate) up to now normally the surgeon could not realize a smooth, gentle and stutter-free (continuous) forward movement with the known instrument since a hooking of the instrument tip could not be excluded on account of the varying bone structure when the same came upon a harder bone area from a softer bone area. Accordingly, in order to obtain a linear cut the instrument tip was not moved continuously forwardly but rather forwardly and backwardly in order to overcome the correspondent obstruction (hard bone structure). Thus, smooth and continuous (stutter-free) linear forward movements without hooking were scarcely realizable, i.e. coming upon harder bone areas resulted again and again in a braking or stagnation of the forward movement so that then the instrument was moved back and once again forwardly with greater pressure in order to cut the hard bone area.

It is the object of the invention to provide a surgical instrument of the cited kind with which an especially smooth and continuous (stutter-free) cutting movement can be carried out.

According to the invention this object is achieved with an instrument of the cited kind by the feature that the rear flank of the cutting teeth extends linearly up to the tooth tip and the leading flank of the same extends in a first portion with large slope from the root of the tooth up to a break point and from there in a second portion with smaller slope as in the first portion to the tip of the tooth located on the other side of the axis of the tooth.

Accordingly, with the instrument of the invention the leading flank of the cutting teeth is provided with a cut or flatter part in the upper portion of each tooth by means of which the corresponding tooth flank is smoothed down. So, the leading flank of each cutting tooth has two portions with different slopes wherein the portion adjacent to the root of the tooth has a larger slope. In contrast to this the rear flank of the cutting teeth extends linearly up to the tip of the tooth and thus has no break point.

When using the instrument a smooth and continuous (stutter-free) forward movement without hooking can be obtained with the inventive design of the cutting teeth. Not only soft but also hard bone areas can be cut uniformly (more easily) so that, when carrying out a linear bone cut, the instrument can be moved substantially uniformly forwardly without having to be moved forwardly and backwardly on account of a braking or hooking.

With the term "forward movement" of the instrument which is used here it is meant that the user of the instrument moves the same away from his body by means of the handpiece. A "backward movement" represents the opposite movement.

The terms "leading flank" and "rear flank" of a cutting tooth define the tooth flank located on the front side and on the back side during a "forward movement" in the above-defined sense.

Normally, the handling of the instrument is realized such that the tip of the instrument points downwardly. However, this does not exclude that a handling is also possible in an opposite manner, i.e. with upwardly directed tip.

In the leading flank of the cutting teeth which is provided with a cut according to the invention the break point is located between the two portions with different slopes preferably at a height corresponding to 0.6-0.7 times, especially 0.64 times, the value of the tooth height. Accordingly, the lower 2/3 of the tooth height are formed by the portion with greater slope while the upper 1/3 of the tooth height is formed by the portion with smaller slope.

Preferably, the surgical instrument described here has an angular instrument shaft so that the shaft portion adjacent to the tip is angled forwardly, i.e. away from the handpiece, when the instrument is handled horizontally with horizontally arranged handpiece. The corresponding angle of the shaft of the instrument is preferably 60°. Of course, with such an angular shaft the second portion with smaller slope is located on the tooth flank which is located on the side of the outer angle of the angle of the shaft. This tooth flank is designated as leading flank.

Practically, the slope of the first portion of the leading flank of the cutting teeth corresponds to the slope of the rear tooth flank. In other words, according to the inventive instrument the tip of the instrument, apart from the cut provided at the leading flank of the cutting teeth, corresponds to the above-described instrument of the prior art according to which both flanks of a cutting tooth have the same slope.

The slope angle of the rear tooth flank with respect to the axis of the tooth preferably has a value of 17.5° which corresponds to the embodiment of the prior art. Accordingly, adjacent tooth flanks of adjacent teeth form together an angle of preferably 35°.

According to the invention the slope angle of the second portion of the leading flank of the cutting tooth has a value of preferably 30-60°, preferably of 45°, with regard to the axis of the tooth. With such a range the best results were obtained with respect to the desired stutter-free forward movement.

Preferably, the tip of the instrument is laterally enlarged with respect to the shaft of the instrument as this is also the case with the instrument of the prior art. Accordingly, the instrument has preferably five adjacent cutting teeth of which each cutting tooth is provided with the inventive cut.

According to the inventive instrument the deepest points of the tooth gaps between the individual cutting teeth are preferably located on a straight line which extends perpendicularly with respect to the axis of the shaft portion adjacent to the tip of the instrument. However, other embodiments according to which the deepest points of the tooth gaps are located on a curved line, especially a circular arc, are also possible.

Preferably, the tip of the instrument is designed flat opposite to the shaft of the instrument which is round, and has practically a thickness of 0.4-0.7 mm, especially of 0.5 mm. For instance, the lateral enlargement of the tip with respect to the shaft is realized by sloped portions.

Preferably, the tip (flattened tip) of the instrument is located in the plane of the axis of the shaft. According to other embodiments the tip is angled with respect to the axis of the shaft laterally.

As already mentioned, the preferred embodiment of the inventive instrument has five adjacent cutting teeth. Other preferred embodiments have four or three adjacent cutting teeth.

The instrument designed according to the invention can be also designated as "bone saw", especially "piezo bone saw". Accordingly, in the dental or oral field of plastic osteotomy techniques, as jawbone crest expansion, distraction and removal of bone blocks, across Le Fort osteotomy to corticotomy and root splitting and separation, nearly all fields of osteotomy can be covered. The use of the instrument is not restricted to the oral or dental field but the same can be also used in other medical fields for cutting bone.

In the following the invention is described by means of an example in connection with the drawing in detail. Of the drawing
- Figure 1: shows a side view of a surgical instrument;
- Figure 2: shows the portion of the instrument contained in circle A of figure 1 in an enlarged representation;
- Figure 3: shows the portion of the instrument contained in circle B of figure 2 in a further enlarged representation;
- Figure 4: shows a longitudinal section through the instrument of figure 1 in a condition turned about 180° with respect to figure 1;
- Figure 5: shows a top view of the instrument; and
- Figure 6: shows a side view of the instrument as in figure 1 in an enlarged condition.

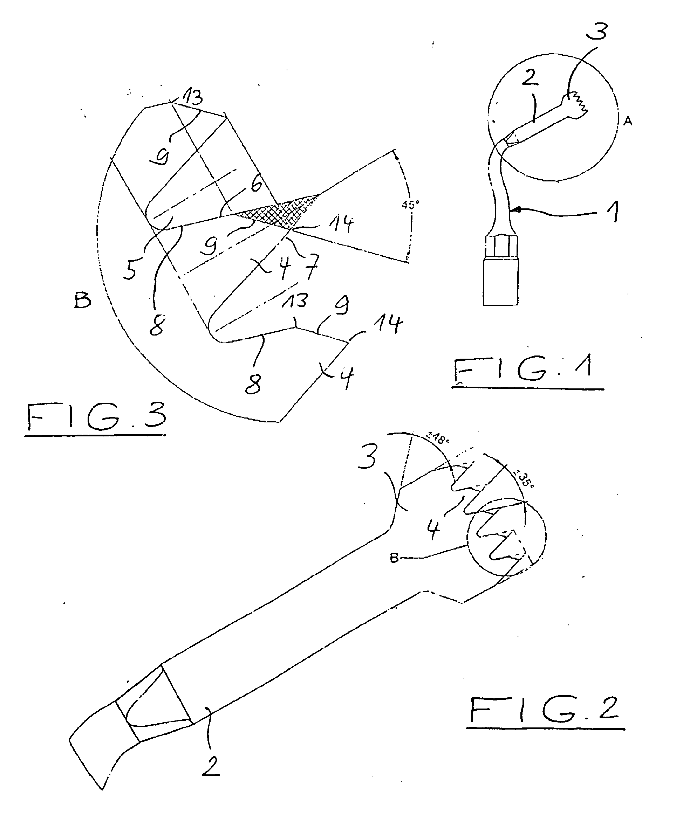

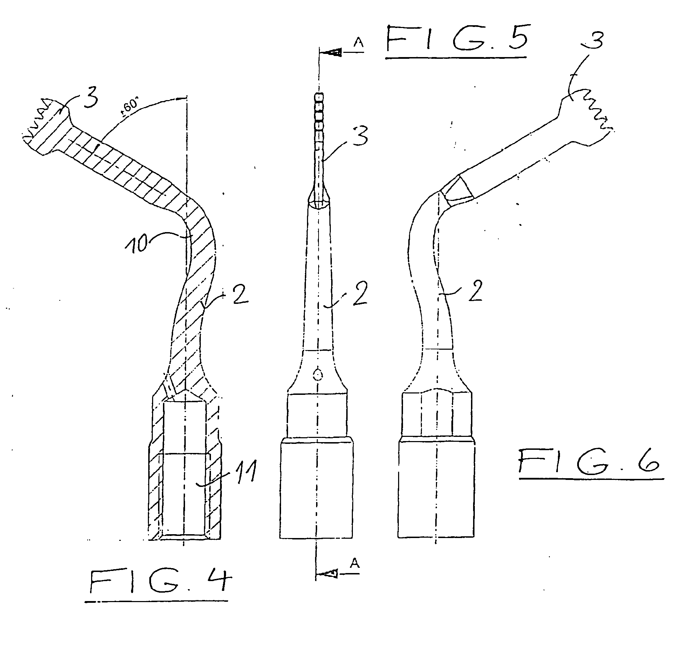

The surgical instrument 1 shown in figure 1 in a side view is an instrument for cutting bone by means of ultrasonics, also designated as piezoelectric bone saw. The instrument is fixed at a handpiece. For this the shaft 2 of the instrument has an enlarged end including a hollow space 11 (shown in figure 4) into which a corresponding connection portion of a handpiece can be inserted. The shaft 2 of the instrument is angularly formed and merges into a tip 3 of the instrument the design of which is relevant for the present invention. The remaining design of the instrument or of the shaft of the instrument can be realized according to the prior art.

Figure 2 shows the tip 3 of the instrument in an enlarged condition. The tip is formed flat and laterally enlarged with respect to the shaft of the instrument which is substantially round wherein two portions angularly formed with respect to the axis of the shaft merge into lateral boundary portions of the tip 3 extending substantially parallel with respect to the axis of the shaft. On its front side the tip has five adjacent cutting teeth 4 which are shown in the excerpt B of figure 3 in larger details.

The surgical instrument 1 shown in the figures is applied with vibrations in the ultrasonic frequency range wherein the tip 3 of the instrument substantially oscillates about the axis of the shaft portion adjacent to the tip. For cutting bone the instrument is moved forwardly by means of the handpiece substantially in a direction perpendicular to the axis of the shaft portion adjacent to the tip so that the flanks shown in figure 3 on the left side of the teeth 4 form leading flanks 6 and the other flanks form rear flanks 7. The leading flanks 6 of the five cutting teeth 4 have a lower portion 8 adjacent to the root of the tooth the slope of which corresponds to the slope of the rear tooth flank 7. Furthermore, the leading flank 6 has an upper portion 9 adjacent to the tip of the tooth which has a smaller slope than the lower portion 8. While the lower portion 8 of the leading flank 6 as well as the rear flank 7 form a slope angle of 17.5° with respect to the axis of the tooth the upper flatter portion 9 has a slope angle of 45° with respect to the axis of the tooth. In figure 3 the tooth portion is shown in a hatched condition which, compared to the embodiment of the prior art, is removed in the embodiment of the invention shown in this figure by a cut or a corresponding slope. The left and right tooth of figure 3 show the leading flanks of the cutting teeth without this hatched portion.

The tooth gaps located between the individual cutting teeth 4 are provided with reference number 5. The respective break point between the lower portion 8 and the upper portion 9 of the leading flank 6 of the cutting teeth has reference number 13.

By means of the inventive cut the tooth tip 14 is no more located at the axis of the tooth but is offset with respect to the same in figure 3 to the right.

With the embodiment shown in figures 1 to 3 the height of a cutting tooth 4 amounts to 0.70 cm wherein the break point is located at a height of 0.45 cm. This substantially corresponds to a value of 2/3 of the height of the tooth.

Figure 4 shows a longitudinal section through the instrument of the figures 1 to 3 in a condition turned around 180°. Here, the tip 3 of the instrument points to the left in the figure. The angled portion of the shaft 2 of the instrument adjacent to the tip forms an angle of 60° with the portion adjacent to the reception end of the handpiece.

Figure 5 shows the instrument 1 in a top view or front view. The tip 3 of the instrument has a thickness of 0.55 mm.

The cut formed on the leading flanks 6 of the individual cutting teeth 4 is located in the representation of figure 4 on the upper side of the teeth, i.e. on the side of the outer angle of the shaft. Accordingly, the forward movement for carrying out a bone cut is realized in figure 4 substantially from above, i.e. parallel with respect to the straight line connecting the deepest points of the tooth gaps or parallel with respect to the portion of the shaft which is not angled.

Figure 6 shows a side view of the instrument as in figure 1 in an enlarged scale.

## Claims

1. A piezoelectric surgical instrument for cutting bone, which is adapted to be fixed at a handpiece, said instrument comprising an instrument shaft adapted to be fixed at the handpiece and a joining instrument tip which has at its free end a plurality of adjacent cutting teeth with two tooth flanks merging into a tooth tip and emanating from a tooth root, **characterized in that** the rear flank (7) of the cutting teeth (4) extends linearly up to the tooth tip (14) and the leading flank (6) of the same extends in a first portion (8) with large slope from the root of the tooth up to a break point (13) and from there in a second portion (9) with smaller slope as in the first portion (8) to the tip (14) of the tooth located on the other side of the axis of the tooth.

2. The piezoelectric surgical instrument according to claim 1, **characterized in that** the break point (13) is located at a height which corresponds to 0.6-0.7 fold, especially 0.64 fold, the value of the height of the tooth.

3. The piezoelectric surgical instrument according to claim 1 or 2 having an angularly formed instrument shaft (2), **characterized in that** the second portion (9) with smaller slope is located at the tooth flank (6) which is located on the side of the outer angle of the shaft angle (10).

4. The piezoelectric surgical instrument according to one of the preceding claims, **characterized in that** the slope of the first portion (8) of the leading flank (6) corresponds to the slope of the rear tooth flank (7).

5. The piezoelectric surgical instrument according to one of the preceding claims, **characterized in that** the slope angle of the rear tooth flank (7) is 17.5° with respect to the tooth axis.

6. The piezoelectric surgical instrument according to one of the preceding claims, **characterized in that** the slope angle of the second portion (9) of the leading flank (6) is 30-60°, especially 45°, with respect to the tooth axis.

7. The piezoelectric surgical instrument according to one of the claims 3 to 6, **characterized in that** the angle (10) of the instrument shaft is 60°.

8. The piezoelectric surgical instrument according to one of the preceding claims, **characterized in that** the instrument tip (3) is laterally enlarged with respect to the instrument shaft (2).

9. The piezoelectric surgical instrument according to one of the preceding claims, **characterized in that** the tooth tip (3) has a thickness of 0.35-0.7 mm, especially of 0.55 mm.

## Patentansprüche

1. Piezoelektrisches chirurgisches Instrument zum Schneiden von Knochen, das an einem Handstück fixiert werden kann, mit einem Instrumentenschaft, der am Handstück befestigt werden kann, und einer sich anschließenden Instrumentenspitze, die an ihrem freien Ende eine Vielzahl von benachbarten Schneidzähnen mit zwei Zahnflanken, die in eine Zahnspitze übergehen und von einem Zahnfuß ausgehen, besitzt, **dadurch gekennzeichnet, dass** sich die hintere Flanke (7) der Schneidzähne (4) linear bis zur Zahnspitze (14) erstreckt und dass sich die vordere Flanke (6) derselben in einem ersten Abschnitt (8) mit großer Steigung vom Fuß des Zahnes bis zu einem Knickpunkt (13) und von dort in einem zweiten Abschnitt (9) mit kleinerer Steigung wie im ersten Abschnitt (8) bis zur Spitze (14) des Zahnes, die sich auf der anderen Seite der Achse des Zahnes befindet, erstreckt.

2. Piezoelektrisches chirurgisches Instrument nach Anspruch 1, **dadurch gekennzeichnet, dass** sich der Knickpunkt (13) auf einer Höhe befindet, die dem 0,6-0,7-fachen, insbesondere 0,64-fachen, Wert der Höhe des Zahnes entspricht.

3. Piezoelektrisches chirurgisches Instrument nach Anspruch 1 oder 2, das einen winklig ausgebildeten Instrumentenschaft (2) aufweist, **dadurch gekennzeichnet, dass** der zweite Abschnitt (9) mit geringerer Steigung auf der Zahnflanke (6) angeordnet ist, die sich auf der Seite des Außenwinkels des Schaftwinkels (10) befindet.

4. Piezoelektrisches chirurgisches Instrument nach einem der vorangehenden Ansprüche, **dadurch gekennzeichnet, dass** die Steigung des ersten Abschnittes (8) der vorderen Flanke (6) der Steigung der hinteren Zahnflanke (7) entspricht.

5. Piezoelektrisches chirurgisches Instrument nach einem der vorangehenden Ansprüche, **dadurch gekennzeichnet, dass** der Steigungswinkel der hinteren Zahnflanke (7) 17,5° relativ zur Zahnachse beträgt.

6. Piezoelektrisches chirurgisches Instrument nach einem der vorangehenden Ansprüche, **dadurch gekennzeichnet, dass** der Steigungswinkel des zweiten Abschnittes (9) der vorderen Flanke (6) 30-60°, insbesondere 45°, relativ zur Zahnachse beträgt.

7. Piezoelektrisches chirurgisches Instrument nach einem der Ansprüche 3 bis 6, **dadurch gekennzeichnet, dass** der Winkel (10) des Instrumentenschaftes 60° beträgt.

8. Piezoelektrisches chirurgisches Instrument nach einem der vorangehenden Ansprüche, **dadurch gekennzeichnet, dass** die Instrumentenspitze (3) relativ zum Instrumentenschaft (2) seitlich erweitert ist.

9. Piezoelektrisches chirurgisches Instrument nach einem der vorangehenden Ansprüche, **dadurch gekennzeichnet, dass** die Zahnspitze (3) eine Dicke von 0,35-0,7 mm, insbesondere von 0,55 mm, besitzt.

## Revendications

1. Instrument chirurgical piézoélectrique pour couper les os, qui est adapté pour être fixé à une pièce à main, ledit instrument comprenant un arbre d'instrument adapté pour être fixé à la pièce à main et une pointe d'instrument de jonction a, à son extrémité libre, une pluralité de dents de coupe adjacentes avec deux flancs de dents fusionnant en une pointe de dent et émanant d'une racine de la dent, **caractérisé en ce que** le flanc arrière (7) des dents de coupe (4) s'étend linéairement jusqu'à la pointe de dent (14) et le flanc avant (6) de celles-ci s'étend dans une première partie (8) ayant une forte inclinaison de la racine de la dent jusqu'à un point de rupture (13) et depuis là dans une seconde partie (9) avec une inclinaison plus faible que dans la première partie (8) jusqu'à la pointe (14) de la dent située de l'autre côté de l'axe de la dent.

2. Instrument chirurgical piézoélectrique selon la revendication 1, **caractérisé en ce que** le point de rupture (13) est situé à une hauteur qui correspond à 0,6 à 0,7 fois, en particulier 0,64 fois, la valeur de la hauteur de la dent.

3. Instrument chirurgical piézoélectrique selon la revendication 1 ou 2, ayant un arbre d'instrument formé de manière angulaire (2), **caractérisé en ce que** la seconde partie (9) avec une inclinaison plus faible est située au niveau du flanc de la dent (6) qui se trouve sur le côté de l'angle extérieur de l'angle de l'arbre (10).

4. Instrument chirurgical piézoélectrique selon l'une des revendications précédentes, **caractérisé en ce que** l'inclinaison de la première partie (8) du flanc avant (6) correspond à l'inclinaison du flanc de dent arrière (7).

5. Instrument chirurgical piézoélectrique selon l'une quelconque des revendications précédentes, **caractérisé en ce que** l'angle d'inclinaison du flanc de dent arrière (7) est de 17,5° relativement à l'axe de la dent.

6. Instrument chirurgical piézoélectrique selon l'une des revendications précédentes, **caractérisé en ce que** l'angle d'inclinaison de la seconde partie (9) du flanc avant (6) est de 30 à 60°, en particulier 45°, relativement à l'axe de la dent.

7. Instrument chirurgical piézoélectrique selon l'une des revendications 3 à 6, **caractérisé en ce que** l'angle (10) de l'arbre de l'instrument est de 60°.

8. Instrument chirurgical piézoélectrique selon l'une des revendications précédentes, **caractérisé en ce que** la pointe de l'instrument (3) est latéralement élargie relativement à l'arbre de l'instrument (2).

9. Instrument chirurgical piézoélectrique selon l'une des revendications précédentes, **caractérisé en ce que** la pointe de la dent (3) a une épaisseur de 0,35 à 0,7 mm, en particulier de 0,55 mm.
